Europäisches Patentamt

European Patent Office

Office européen des brevets

(19)

(11) Publication number: **0 232 235**
A2

(12) **EUROPEAN PATENT APPLICATION**

(21) Application number: 87850019.8

(22) Date of filing: 23.01.87

(51) Int. Cl.³: **A 61 M 15/00**

(30) Priority: 27.01.86 DK 389/86
09.04.86 DK 165/87
06.05.86 DK 2076/86
01.10.86 DK 4675/86

(43) Date of publication of application:
12.08.87 Bulletin 87/33

(84) Designated Contracting States:
ES GR

(71) Applicant: Aktiebolaget Draco
Box 34
S-221 00 Lund(SE)

(72) Inventor: Holm, Karl
Rudesvej 4
Dk-7500 Holstebro(DK)

(74) Representative: Hjertman, Ivan T. et al,
AB ASTRA Patent and Trade Mark Department
S-151 85 Södertälje(SE)

(54) Medical dosing device for discharge of atomized medicament for inhalation air.

(57) The invention relates to a medical dosing device for discharge of medicament for inhalation. The device comprise a handheld holder (2) for a medicine container (28;40) from which medicine is discharged via a valve (18;50;82) into an air channel (6) for inhalation air by means of initiation of an activation device (12;94). The valve is operationally connected with a control unit (14;90) arranged, on initiation of the activation device (12;94), to control the discharge valve (18;50;82) for intermittent opening and closing repeatedly within an inhalation period. The control unit is preferably an electronically controlled unit (14) which activates an electrically controlled discharge valve (18;50).

FIG. 6

EP 0 232 235 A2

0232235
87-01-16

The present invention relates to a medical spray device of the type that consists of a handheld holder for a medicament container from which atomized medicament may be sprayed via a nozzle by operation of an actuation device. Such devices are used by patients with respiratory diseases, in that the atomized medicament is inhaled, for example via an inhalation mouthpiece on the holder.

The most common devices of this type are based on the use of medicament containers in which the medicament is mixed with a propellant gas in liquid form. This mixture is dispensed in the form of an aerosol by depressing the valve on the aerosol. Normally when it is desirable to avoid an excessive dose, aerosols of the type in which depression of the valve only results in the discharge of a single metered dose of medicament are used. In this way, it is possible to prescribe the number of doses the patient should inhale on each occasion the device is used.

In practice, the patient presses the aerosol container in towards a junction piece for the valve in the holder thereby activating a dosing valve in the aerosol. The dose discharged flows via a short pipe from the junction piece to the spray nozzle, which is often merely a simple endpiece for this pipe. Typically, the dose delivered is seen as a brief, substantial discharge, spreading in a fan from the nozzle opening.

It has been found that in this process, extremely inefficient use is made of the medicament, in that, during the substantial discharges, the gas/medicament mixture is not atomized efficiently and a considerable amount of the mixture is thereby deposited in liquid form in the areas of the mouth and throat coming into contact with the sprayed mixture, while only a small amount is atomized finely enough to permit the propellant gas to vaporize immediately and leave the medicament behind as fine particles which can be carried along with the inhaled air current.

However, it has come to be accepted that, although the simple handheld

devices referred to here are not particularly efficient, they do have the advantage of being so simple and inexpensive that they are obtainable and usable by anyone.

Devices of the "fixed-position" type normally referred to as nebulizers are known, for example for hospital use. In such devices e.g. an airstream is passed through an area in which the medicament is introduced, allowing the latter to be atomized. It is an advantage for the medicament to be purely water based, and for a propellant – which is not exactly a medically desirable ingredient in inhalation air – not to be used. Such devices, which thus need a high-pressure air supply, among other requirements, do not thus represent an alternative to patients own simple handheld devices for domestic use.

The object of the invention is to provide a device of the type described latterly above, and thus also in the introduction to the above, which, despite being a simple handheld device is nevertheless able to utilize the medicament far more efficiently than hitherto.

The invention is based primarily on the observation that the medicament may be utilized far more efficiently if, during the various inhalation processes, it is dosed by an automatically controlled intermittent method, such that within this period not just a single large dose of medicament is discharged, but a series of rather small doses. According to the method of atomization used, this will provide to a greater or lesser extent a pronounced increase in the amount of small particles of the medicament supplied in each inhalation. Not only is it possible for the small doses generally to be atomized more efficiently than one large dose, the process of atomization can also be achieved more quickly. This results in a dose with a particle size distribution which allows for a better distribution and thereby a better therapeutic effect in the lung.

A reduction of the fraction of the dose deposited in the mouth and throat is also important as it does not produce any therapeutic effect but only increases the risk for unwanted local and systemic side effects.

The invention is thus characterized primarily by the features stated in the characterizing clause of claim 1. The dosing device according to the invention will, for understandable reasons, be more expensive than the devices known hitherto; however, the necessary increase in cost is quite small in relation to the increase in efficacy and thereby reduction in consumption in medicament offered.

As stated in claim 4, it is preferred that the valve should be controlled in such a way that it will open for time periods of 2-5 milliseconds, while the intervals between these periods will preferably be 50-200 milliseconds. Thus, in practice, the valve will be operated in such a way as to discharge, for example, 5-15 rather small doses in the course of 1-3 seconds, in that inhalation periods substantially in excess of that period ought not to be considered. Given the very short "open-aperture" periods, a fairly large nozzle aperture may be used, thus avoiding blockages.

The valve may suitably be in the form of a solenoid valve actuated by a battery incorporated into the device via a simple electronic control unit to determine the intermittent function of the valve. However, the valve may also be operated by other means, including mechanical, which are explained in greater detail in the following.

In the invention, it is further recognized that, in order to achieve satisfactory efficiency for the device, it should be ensured that the distance should be kept very short between the area in the valve in which the medicament is under pressure and the nozzle aperture itself from which the medicamet is sprayed when the valve is opened. When relatively large doses are discharged, this factor is not particularly important, and it is also common for outlet pipes of considerable length to be found between the outlet junction on the medicament container and the spray discharge nozzle on the device. In the event that such a relatively long pipe connection is used, a considerable amount of the energy for the discharge will be consumed in building up a discharge pressure in the pipe connection itself; similarly, on completion of the discharge, there will be a phase in which the pressure in this pipe connection will be falling in such a way that a certain proportion of the gas/medicament mixture will hardly be discharged with any sufficient atomization effect. This may be illustrated by the fact that a discharged dose of quite microscopic size would not reach even as far as the nozzle aperture for spraying with any useful atomization effect, while in the case of a large dose it is of no particular significance whether there are relatively short periods at the beginning and end during which atomization is ineffective.

In order to further increase the efficiency in the use of the medicament, it is thus beneficial for the device to be equipped as described in claim 6, where the short distance referred to is essential if efficient atomization of the medicament is to be achieved during the opening and closing movements of the valve, with the result that the minimum possible medicament is wasted during the fairly brief openings of the valve.

Provided that the short discharges are efficiently atomized the mixture of medicament and evaporating gas is normally removed continously by the inhalation air flow before the next discharge, which will prevent the medicament build up in the discharge area. Sufficient removal of discharged aerosol will take place even at very low inhalation flow rates.

In principle, an aerosol may be used with a dosing valve that, for each operation, delivers a considerable smaller quantity than hitherto, and that which is activated repeatedly by automatic means; however it will be preferred that a separate valve forming part of the holder will be used with the result that the aerosol may accordingly be equipped with a simple discharge valve actuator through which the valve may be permanently supplied with the aerosol fluid at full pressure.

However, the manner by which the necessary propellant pressure is applied is not crucial to the invention. Thus, an excellent arrangement would be a medicine container with a water-based medicament in which, by means of compressed air or some other means, it could be ensured that the liquid is delivered to the valve at such a pressure as is required to achieve efficient atomization during spraying from the valve. In the following, a combination of solenoid valve and atomization nozzle specially developed for the invention is described, which combination is particularly well-suited for efficient atomization of a water-based medicament during the brief openings of the valve.

The invention is described in more detail in the following text, with references to the drawings, in which:

Figure 1 is a sectional view of a first embodiment of the invention;

Figure 2 is a top elevation of the same;

Figure 3 is a graphical representation of the device's intermittent function;

Figure 4 is a sectional view of a second embodiment of the invention .

Figure 5 is a similar representation of a third embodiment of the invention;

Figure 6 is a sectional view of a fourth embodiment of the invention;

Figure 7 is a front elevation of the same;

Figure 8 is a sectional view of a fifth embodiment of the invention;

Figure 9 is an enlarged view of the valve housing in Figure 8;

Figure 10 is a front elevation of Figure 8.

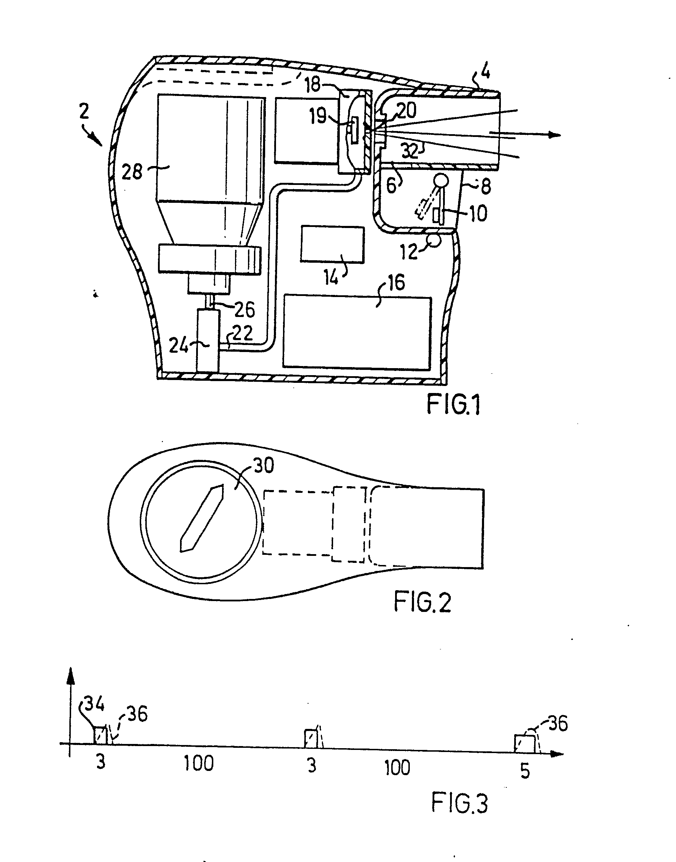

The device illustrated in Figures 1 and 2 consists of a housing 2 for the device with a projecting tubular mouthpiece 4, which continues into an internal air channel 6 connected to an air intake opening 8 below the tubular mouthpiece 4. A inwardly-hinged locking flap 10 is incorporated inside the opening 8, on which flap 10 is fitted a small magnet and which when opened as a result of suction on mouthpiece 4 activates a Reed contact 12 to transmit a signal to an electronic control unit 14 with suitable battery 16.

A solenoid valve 18 with a discharge opening 20 pointing axially and centrally out into mouthpiece 4, is fitted on the rear wall of air channel 6. A pipe 22 to the solenoid valve 18 is connected with a junction piece 24 to connect with a discharge valve connector 26 on an aerosol 28 which is inserted via an opening in the top of housing 2 and is fitted with the upward facing part of its base in contact with a stop or press-on lid 30 in such a way that the aerosol is held down under pressure to keep permanently open the built-in discharge valve at the valve connector 26, that is, such that after fitting of the aerosol 28 pressurized liquid will be permanently supplied to the inlet of the solenoid valve 18. The aerosol contains a mixture of medicament and liquid propellant gas in the customary way. It is not customary, in a medical aerosol to be used in inhalation devices, for a simple aerosol valve to be used for permanent activation when the valve 26 is depressed, in that it has been customary for a dosing valve to be used which delivers a certain dose of liquid at each depression of the valve. In the device according to the invention, a medical aerosol of a simplified but otherwise well-known design may thus be used.

After insertion of the aerosol 28, a constant pressure will thus be applied to the solenoid valve 18, and this valve may be activated into opening for spraying a thin and therefore quickly vaporizing jet of liquid 32 into the mouthpiece 4 in response to the user of the device putting his/her mouth to the mouthpiece 4 and inhaling strongly enough to activate the air flap 10. The jet of medicine 32 from the valve nozzle aperture 20 will be directed into the user's throat, in any event, and may even in this respect provide a therapeutic benefit which may rapidly increase the patient's ability to achieve a yet more efficient inhalation.

The invention permits a reasonably large nozzle aperture 20 to be used, and thus offers good reliability, in that it is ensured by an electronic control unit 14 that the solenoid valve 18 is activated in such an intermittent manner that the medicament is delivered in rather small doses, as described above.

As illustrated in Figure 3, it is possible, for example, for the control unit 14 to transmit an "open" pulse 34 with a duration of about 3 milliseconds to the solenoid valve 18, which - shown as a dotted line 36 - immediately begins to open, whereas it hardly completes the opening process before the open pulse is broken off, that is, the valve will close immediately after, or even before it has opened completely, depending on the reaction speed of the valve. About 100 milliseconds after that an open pulse is again transmitted, and this process continues while a predetermined number of doses is automatically counted, or for a predetermined period of time of 1-3 seconds.

On the far right of Figure 3 it is shown that a pulse period of 5 milliseconds may be applied, and that in such a case the solenoid valve may open fully: this is represented by the flattened peak of curve 36 for the duration of this period.

The control unit may be designed as adjustable, such that the pulse period and/or repetition period may be adjusted as required, either by the user him or herself or preferably at the laboratory adjustment stage; similarly it will also be possible to adjust the number of pulses or the total operating period after activation.

Furthermore, the control unit should also be equipped so that reactivation may not take place within a certain period of time after a period during which the device has been operated, for example, within the 10-60 seconds immediately thereafter since the patient otherwise may risk an accident overdose.

As mentioned above, it is essential for the distance to be kept very short between the valve seat of the solenoid valve and the aperture in the spray nozzle from which the medicament/gas mixture emerges, fully reduced to atmospheric pressure, and it will be seen that this requirement is satisfied in the illustrated embodiments, where the valve seat is located immediately adjacent to the inner end of the aperture 20, in that the valve seat cooperates with a valve body in the form of a plate 19 moved by an electromagnetic system (not shown) and by a return spring.

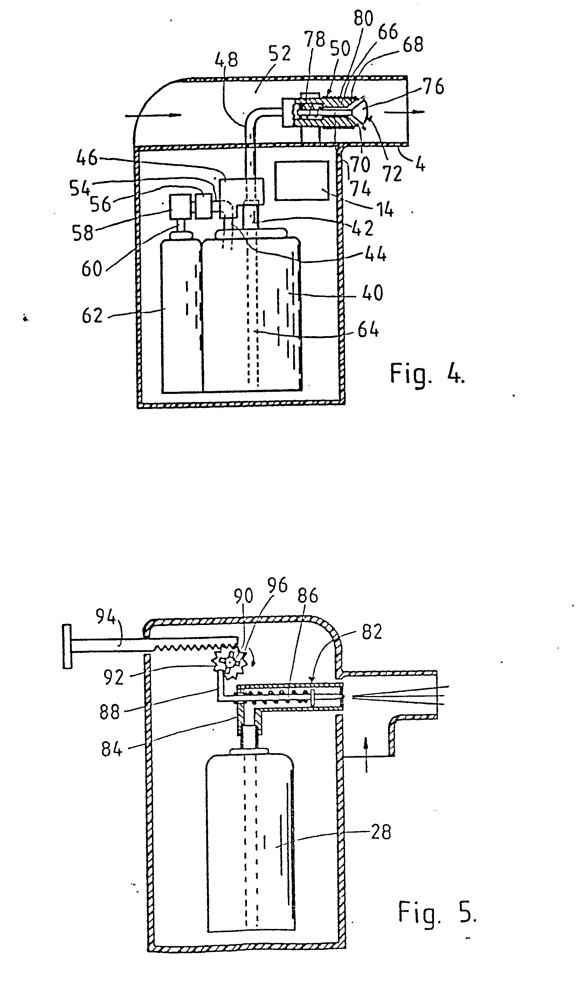

In the embodiment illustrated in Figure 4, a medicament container 40 is used that contains a water-based medicament, and that is fitted with both an outlet connector 42 and an inlet connector 44 for compressed air for the space above the liquid contained in the container 40. The device incorporates a junction piece 46 for the two connectors, in that the outlet connector is brought by this means into contact with a inlet pipe 48 for supply of a water-based medicament to a atomization valve 50 into an air passage 52 and to an inhalation mouthpiece 4, while the connector 44 is brought into contact with a branch pipe 54, which is connected via a pressure regulator unit 56 to a separate junction piece 58. It is anticipated that the latter will accommodate an outlet valve connector 60 on a small compressed air container 62, which can be inserted into the device at the same time as a new medicament container 40 in order to enable the water-based medicament in the container 40 to be expelled from the container, from a riser pipe 64 emerging from the connector 42, at an essentially constant pressure, until the container is entirely empty.

Alternatively, at the top of the container 40, a compressed air chamber may be provided which is of sufficient size to maintain a reasonably high propellant pressure on the liquid in the container 40 throughout the period during which the container is being emptied. Otherwise the pressure regulator 56 will ensure that an exactly defined propellant pressure on the medicament liquid is maintained throughout the period.

The exhaust valve 50 is constructed in the form of a combined solenoid valve and exhaust nozzle, in that it consists of a cylindricl valve housing 66, the rear of which is connected to the discharge pipe 48 and the front of which incorporates a conical valve seat 68 including a seat ring 70 at its outer end. A suitable valve body 72 consists of a central rod 74 of a magnetic material and a conical valve head 76 at the front. The valve incorporates a spring which normally holds the valve head locked against the seat ring 70. Around the front part of the valve housing 66, an electric coil 80 is arranged, which, on activation from the control unit 14, moves the central rod 74 forward to open the valve.

As the valve opens, the water-borne medicament is sprayed out in a fan-formed, very thin layer, which allows for a rapid and efficient atomization of the medicament, such that the latter may be driven along by the inhalation air.

Figure 4 contains no illustration of any activation device for the control unit 14, but a manual activation button or sensor device may be used to detect an inhalation air stream, possibly on the principle illustrated in Figure 1.

In the embodiment illustrated in Figure 5, a purely mechanical control unit is used in combination.with a float needle valve 82 and an aerosol 28 whose discharge connector is accommodated by an inlet junction piece 84 on the valve 82. The float needle 86 on the latter emerges via an airtight seal from the rear of the valve, and incorporates a hooked rear end 88 which cooperates with a cam 90. This cam is linked via a one-way drive mechanism to a ratchet wheel 92 which can be rotated by means of a rack 94 which can be pressed in from the outside against the pressure of a return spring (not shown). As the rack 94 is pressed in, both the ratchet wheel 92 and the cam 90 are rotated, on which the notches 96 activate the hooked end 88 and thus the valve needle 86 into performing a series of brief openings of the valve. The return movement of the rack only rotates the ratchet wheel.

Another possibility is to use a form of control in which the rack is under passive pressure to tension the return spring, after which the valve is activated when the user releases or triggers the rack; a simple "moderating" brake, such as a rotating air brake, which ensures a suitably low speed of movement in the system could be incorporated.

It is possible that a suitable valve could be activated through the use of an electric motor to turn the cam 90. The motor should preferably be time-controlled so that each time it is activated it operates only for the required brief period of time within the inhalation period; however, there will also be patients who are themselves able to control activation and thus merely start and stop the motor by a simple control circuit breaker.

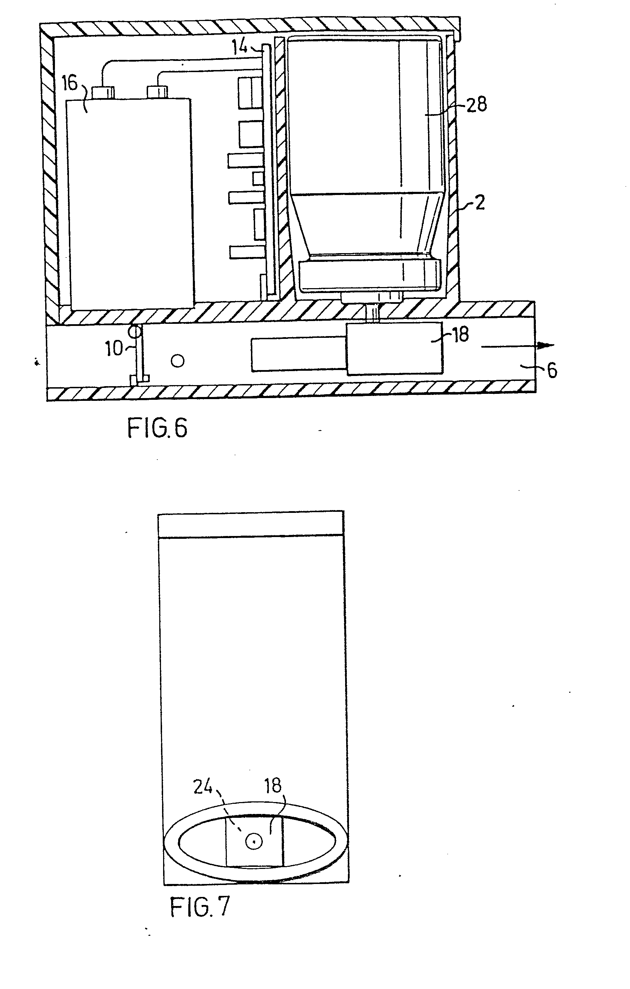

Figures 6 and 7 illustrates an embodiment which is a modification of the device shown in Figure 1, similar reference numerals being used. The device consists of a housing 2 with a projecting tubular mouthpiece, which continues into an internal air channel 6 connected to an air intake opening. A inwardly-hinged locking flap 10 with a magnet is placed inside the air intake opening, which flap 10 when opened, as a result of suction on the mouthpiece, activates a Reed contact to transmit a signal to an electronic control unit 14 with a battery 16. A junction piece 24 for the valve connector 26 of the aerosol 28 is mounted in direct association with a solenoid valve 18. The combination of the junction piece 24 and the solenoid valve 18 is incorporated inside the air channel and said solenoid valve is controlled to discharge a predetermined number of small part-doses from the aerosol container 28 within one inhalation. The aerosol is held down under pressure in the housing 2 to keep the built-in discharge valve permanently open.

This embodiment shows a device with a short distance between the aerosol container 28 and the nozzle aperture, such that a connector pipe 22 is avoided. This short distance is essential for efficient atomization of the medicament while the minimum possible medicament is wasted during the brief openings of the valve.

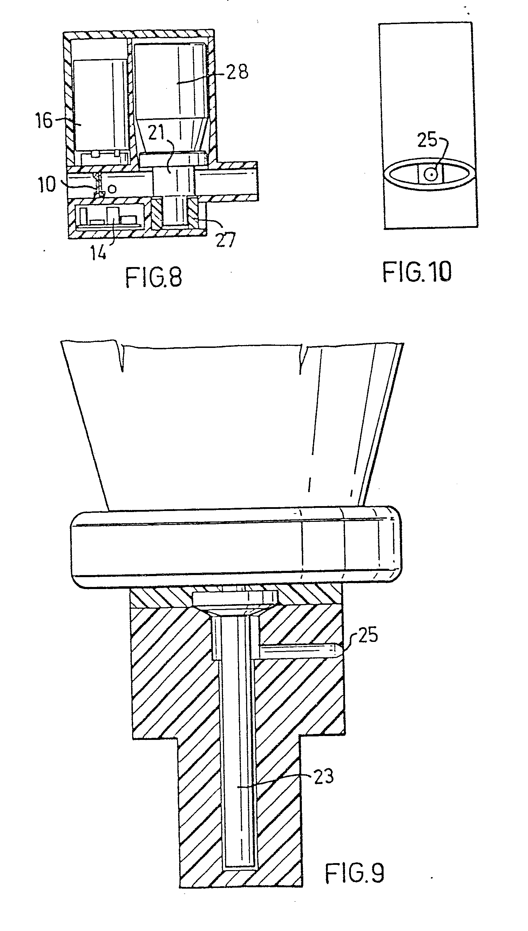

Figures 8, 9 and 10 illustrates an embodiment which is a modification of the "short distance" design shown in Figure 6, why similar reference numerals are being used. This embodiment is adapted for use with a specially designed replaceable aerosol unit, which is shown in the enlarged view of Figure 9. The aerosol unit has a narrowed lower portion containing a valve housing 21 with a valve nozzle aperture 25 and holding a valve body 23 of a magnetic material having at its end a valve plate. In the lower part of the holder, below the air channel an upwardly projecting solenoid 27 is mounted, which solenoid will receive the cylindrical portion of the aerosol unit when the latter is inserted into the holder. Consequently the valve assembly is ready for actuation when the unit is inserted.

By using this type of valve/aerosol container closure the ordinary built-in aerosol valve will be unnecessary and furthermore the "short-distance" design is obtained. The number of components included in the inhalator is reduced and will make the manufacturing more simple and inexpensiv and will also increase the reliability in operation. As a consequence of the separation of the solenoid and the valve body, the aerosol unit can not be activated prior to the introduction into the holder, which prevents leakage of the mixture of medicament and liquid propellant gas. Leakage is a customary problem for an aerosol container with an ordinary discharge valve.

The invention also comprises other containers prepared with integrated particular means for enabling an operation of the entire device in accordance with the principles of the present invention. Thus, the container could also comprise the battery required for the functioning of the device, whereby a fresh battery is provided automatically each time a new container is mounted into the holder.

In the embodiments illustrated in Figure 1, Figure 4, Figure 6 and Figure 8 optimization in the discharge conditions is striven for, but it should be emphasized that even less optimal solutions could bring about considerable improvements in relation to customary dosing technology. The very splitting up of the customary single doses into a number of smaller part-doses within an inhalation will represent an improvement in itself. Similarly, it may be possible to operate with considerably longer "open-aperture" times and shorter "closed-aperture" times than illustrated in Figure 3. In this context, it will be possible to use a vibrating valve or nozzle which, on receiving and discharging the pressurized liquid, is made to vibrate in such a way that the liquid is sprayed intermittently. The control unit mentioned in the above, which is necessary to provide the intermittent function, should not necessarily be an external unit relative to the valve.

It should be mentioned that in designing the optimizing arrangements in connection with aerosols, account should be taken of the fact that the pressure in the aerosols is dependent on temperature, in which case the open-aperture times normally used in the valves may be insufficient at low temperatures. This problem may be overcome simply by using an electronic control unit that incorporates a temperature-sensitive component which is made to effect an automatic adjustment of the valve's open-aperture time, such that the latter is increased in the event of falling temperatures. This can typically be achieved by the use of preprogrammed microchips. Alternatively, a reduction of the closed-aperture time between the successive open pulses can be effected, such that within a given total operating period, an increased number of part-doses will be delivered in the event of falling temperatures. This solution is, however, not by any means the best one, in that in order to ensure efficient use of the medicament it is advantageous to ensure that a vaporized part-dose has been removed from the atomization area by means of the inhalation air current before the next part-dose is emitted. It is for this very reason that the closed-aperture time between the part-doses should preferably not fall below 100 milliseconds, although even at closed-aperture times of 30-50 milliseconds it is possible to achieve reasonably good results.

CLAIMS

1. Medical dosing device for discharge of medicament, for inhalation, said device comprising a medicine container (28; 40) from which medicine is discharged via a valve (18; 50; 82), characterized in that said valve (18; 50; 82) is arranged to intermittently open and close within one inhalation.

2. Device according to claim 1, characterized in that said salve (18; 50; 82) is activated by a control unit (14; 90), which is adjusted, adjustable or computer controlled for opening and closing of the valve (18; 50; 82).

3. Device according to claim 1, characterized in that said control unit (14; 90) is arranged to activate the valve for discharging 3-30 doses of medicine within a period of 0.5-5 seconds, and preferably 5-15 doses within a period of 1-3 seconds.

4. Device according to claim 2, characterized in that said control unit (14; 90) is arranged to activate the valve for opening periods of 1-50 milliseconds at intervals of 10-500 milliseconds, preferably for 2-10 milliseconds and 50-400 milliseconds, respectively, and specifically for 2-5 milliseconds and 50-200 milliseconds, respectively.

5. Device according to claim 2, characterized in that the control unit (14) is an electrically or electronically controlled unit which activates a directly or indirectly electrically controlled discharge valve.

6. Device according to claim 2, characterized in that the medicin is discharged via a nozzle aperture located in the immediate vicinity of the valve seat area for the valve (18), said nozzle aperture (20) preferably consists of the discharge aperture for the valve (18).

7. Device according to claim 2, characterized in that an inlet channel (22) to said valve (18) is permanently connected with a junction piece (24) to provide an airtight seal with an outlet connector (26) on an aerosol container (28) of the type in which the outlet is opened permanently with pressure being applied to the outlet connector.

8. Device according to claim 2, characterized in that said valve (50) is constructed integrally with a diffusion nozzle, that said valve (50) comprises a valve body (72) with a central rod (74) and a conical valve head (76), said valve body (72) is arranged to slide to and from a sealing contact with the wall of a surrounding conical discharge aperture (60), preferably by electromagnetic activation.

9. Device according to claim 8, characterized in that said valve (50) is surrounded by a .... channel (52) supplying .... ....

10. Device according to claim 9, characterized in that said device comprises a medicine container (40), containing a water-based medicine, and a compressed air container (63), providing the propellant pressure to the medicine container (40).

11. Device according to claim 1, in which said valve (18; 50; 82) is activated by manual initiation of an activation device (12, 94) or preferably by automatic operation as a function of a detected inhalation air stream, characterized in that the control unit (14; 96) is arranged to start the activation process for said valve in response to prior initiation of the activation device, within a period of 0.1-0.5 seconds and similarly, the control unit is arranged so that said valve can only be reactivated a predetermined time after completion of a previous activation process, for example 10-60 seconds thereafter.

12. Device according to claim 5, said device being a handheld inhalator with an aerosol container (28), characterized in that said valve is a solenoid valve (18), which is mounted in direct association with a junction piece (24), said junction piece (24) provides an airtight seal with an outlet connector on said aerosol container (28) of the type in which the outlet is opened permanently with pressure being applied to the outlet connector.

13. Device according to claim 5, said device being a handheld inhalator with an aerosol container, characterized in that said container is integrated with a valve housing (21) holding a valve body (23) and with a nozzle, constituting an integrated replaceable unit and that said valve can only be activated when said unit is introduced into the inhalator.

14. Device according to claim 13, characterized in that said valve is a solenoid valve, that the solenoid (27) is mounted in the inhalator and is arranged to surround said valve housing when said unit is introduced into the inhalator.

15. A medicine container unit for use in a device according to claim 1, characterized in that said unit comprises an aerosol container integrated with a valve housing (21), holding a valve body (23) and with a nozzle, said valve body (23) is activated from the outside by magnetic forces, to effect an intermittent opening and closing of said valve.

16. Method for administering a medicament by inhalation, characterized in that a unit dose of the medicament is administered by intermittent discharging of medicament within one inhalation.

FIG.1

FIG.2

FIG.3

1987-01-23
0232235

Fig. 4.

Fig. 5.

FIG.6

FIG.7

16

10

21

28

14

27

FIG.8

25

FIG.10

25

23

FIG.9